(19) Europäisches Patentamt
European Patent Office
Office européen des brevets

(11) Publication number: **0 397 455 A1**

(12) # EUROPEAN PATENT APPLICATION

(21) Application number: 90304953.4

(51) Int. Cl.[5]: C07D 249/18

(22) Date of filing: 08.05.90

(30) Priority: 08.05.89 US 348533

(43) Date of publication of application:
14.11.90 Bulletin 90/46

(84) Designated Contracting States:
AT BE CH DE DK ES FR GB GR IT LI LU NL SE

(71) Applicant: CALGON CORPORATION
Route 60-Campbell's Run Road
Robinson Township Pennsylvania 15205(US)

(72) Inventor: Vanderpool, Daniel P.
109 Shippen Drive
Coraopolis, PA 15108(US)

(74) Representative: Hesketh, Alan, Dr. et al
European Patent Department Merck & Co., Inc. Terlings Park Eastwick Road
Harlow Essex, CM20 2QR(GB)

(54) **Novel alkoxybenzotriazole compounds.**

(57) Novel alkoxybenzotriazole compounds are disclosed.

EP 0 397 455 A1

## NOVEL ALKOXYBENZOTRIAZOLE COMPOUNDS

### BACKGROUND OF THE INVENTION

Benzotriazole, mercaptobenzothiazole and tolyltriazole are well known copper corrosion inhibitors. For example, see U.S. patent 4,675,158 and the references cited therein. Also, see U.S. patent 4,744,950, which discloses the use of alkylbenzotriazoles as corrosion inhibitors and U.S. patent 4,406,811, which discloses the use of benzotriazole/tolyltriazole blends in water treatment compositions for multimetal corrosion inhibition. While the compound 5-methoxybenzotriazole (anisotriazole) is known and has been disclosed for use in corrosion inhibition compositions (see Japan Kokai Tokkyo Koho, JP 59,222,589; 14 Dec. 1984; Chem. Abst. 102:153153b.), $C_3$, $C_4$, $C_5$, $C_7$ and $C_9$ alkoxybenzotriazoles are not known. These compounds have utility as corrosion inhibitors, particularly copper corrosion inhibitors.

### DESCRIPTION OF THE INVENTION

The instant invention relates to novel compounds having the following structure:

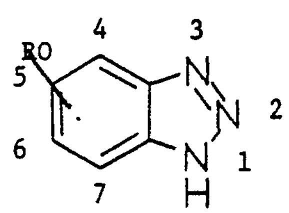

wherein R selected from the group consisting of $C_3$, $C_4$, $C_5$, $C_7$ and $C_9$ alkyl.

The inventor has discovered novel alkoxybenzotriazoles. These compounds have efficacy as corrosion inhibitors, particularly copper corrosion inhibitors. More particularly, these compounds form durable, long-lasting films on metallic surfaces, including but not limited to copper and copper alloy surfaces. The instant alkoxybenzotriazoles are especially effective inhibitors of copper and copper alloy corrosion, and can be used to protect multimetal systems, especially those containing copper or a copper alloy and one or more other metals.

The instant invention also relates to isomers of the above described 5-alkoxybenzotriazoles. The 5 and 6 isomers are interchangeable by a simple prototropic shift of the 1 position hydrogen to the 3 position and are believed to be functionally equivalent as corrosion inhibitors. The 4 and 7 isomers are believed to function as well as or better than the 5 or 6 isomers as corrosion inhibitors, though they are more difficult and expensive to manufacture. As used herein, the term "alkoxybenzotriazoles" is intended to mean 5-alkoxybenzotriazoles and 4, 6 and 7 position isomers thereof.

The instant alkoxy benzotriazoles may be prepared by contacting a 4-alkoxy-1, 2-diaminobenzene with an aqueous solution of sodium nitrite in the presence of an acid, e.g., sulfuric acid, and then separating the resultant oily product from the aqueous solution. The 4-alkoxy-1,2-diaminobenzene may be obtained from any number of sources. The 4-alkoxy group in the precursor 4-alkoxy-1, 2 diaminobenzene used to synthesize an alkoxybenzotriazole is chosen based on the alkoxy group desired in the alkoxybenzotriazole. For example, a 4-pentoxy-1, 2-diamino benzene precursor yields an oily mixture which contains pentoxybenzotriazole.

The instant compounds can be used as water treatment additives for industrial cooling water systems, gas scrubber systems or any water system which is in contact with a metallic surface, particularly surfaces containing copper and/or copper alloys. They can be fed alone or as part of a treatment package which includes, but is not limited to, biocides, scale inhibitors, dispersants, defoamers and other corrosion inhibitors. The instant alkoxybenzotriazoles and substituted alkoxybenzotriazoles can be fed intermittantly or continuously.

### EXAMPLES

The following examples demonstrate the synthesis of the instant compounds. They are not, however, intended to limit the scope of the invention in any way.

5-pentyloxybenzotriazole:

194 g of 4-pentoxy-1,2-diaminobenzene was mixed with 69 g of sodium nitrite in 103 g water and 60 g acetic acid in a glass flask equipped with an agitator, and the reagents were allowed to react. The resulting organic layer was found to contain pentyloxybenzotriazole by proton-NMR spectral analysis.

The 4-pentoxy-1, 2-diamino benzene used was obtained from the following sequence of reactions: 4-pentoxyaniline was reacted with acetic anhydride resulting in 4-pentoxyacetanilide, which was reacted with nitric acid to yield 2-nitro-4-pentoxyacetanilide. This was reacted with dilute acid to give 2-nitro-4-pentoxyaniline, which was reduced in the presence of molecular hydrogen and Raney-nickel catalyst resulting in 4-pentoxy-1, 2-diamino benzene. The 4-pentoxyaniline starting material is commercially available from Aldrich Chemical Company, Inc.

## Claims

1. A compound having the following structure:

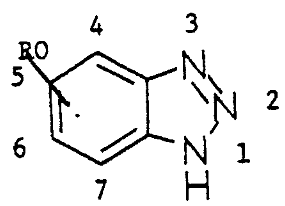

where R is selected from the group consisting of $C_3$, $C_4$, $C_5$, $C_7$ and $C_9$ alkyl. and wherein RO is positioned at 4, 5, 6 or 7.

European Patent Office

# EUROPEAN SEARCH REPORT

Application number

EP 90304953.4

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| X | CHEMICAL ABSTRACTS, vol. 88, no. 18, May 1, 1978 Columbus, Ohio, USA ADACHI, KEIICHI et al. "Benzotriazole deruvatives" page 71, column 2, abstract-no. 122 674k & Jpn. Kokai 77 93,771 | 1 | C 07 D 249/18 |
| A | DE - A1 - 2 641 964 (FUJI) * page 63, line 20 * | 1 | |
| A | CHEMICAL ABSTRACTS, vol. 104, no. 24, June 16, 1986 Columbus, Ohio, USA HORII, MATSUICHI et al. "Method of processing silver halide photographic materials" page 590, column 2, abstract-no. 216 379u & Jpn. Kokai Tokkyo Koho JP 60,154,253 | 1 | TECHNICAL FIELDS SEARCHED (Int Cl 5) C 07 D 249/00 |
| A | CHEMICAL ABSTRACT, vol. 86, no. 22, May 30, 1977 Columbus, Ohio, USA NAKAMURA, NORIYUKI "Polyamide fibers" page 71, column 1, abstract-no. 156 937x & Japan. Kokai 77 22,049 | 1 | |

The present search report has been drawn up for all claims

| Place of search | Date of completion of the search | Examiner |
|---|---|---|
| VIENNA | 16-07-1990 | HAMMER |

CATEGORY OF CITED DOCUMENTS

- X : particularly relevant if taken alone
- Y : particularly relevant if combined with another document of the same category
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document

EPO Form 1503 03 82